# EUROPEAN PATENT SPECIFICATION

(11) **EP 2 939 691 B1**
(45) Date of publication and mention of the grant of the patent: **20.02.2019**
(21) Application number: 13823919.9
(22) Date of filing: 16.12.2013
(51) Int. Cl.: A61K 39/12, C07K 14/005

(54) **DENGUE VIRUS VACCINE COMPOSITION**
VAKZINE ZUSAMMENSETZUNG GEGEN DENGUEVIRUS
COMPOSITION VACCINALE CONTRE LE VIRUS DE LA DENGUE

(30) Priority: 27.12.2012 CU 20120179
(43) Date of publication of application: 04.11.2015
(73) Proprietor: Centro De Ingeniería Genética Y Biotecnología, 11600 La Habana (CU)
(72) Inventor: HERMIDA CRUZ, Lisset, 17100 La Habana (CU); GIL GONZÁLEZ, Lázaro, 33100 Mayabeque (CU); IZQUIERDO OLIVA, Alienys, 32200 Artemisa (CU); MARCOS LÓPEZ, Ernesto, 11500 La Habana (CU); SUZARTE PORTAL, Edith, 10900 La Habana (CU); GUILLÉN NIETO, Gerardo, Enrique, 10400 La Habana (CU); GUZMÁN TIRADO, María, Guadalupe, 11300 La Habana (CU); VALDÉS PRADO, Iris, 10700 La Habana (CU); LAZO VAZQUEZ, Laura, 10700 La Habana (CU); GARCÍA ARECHAVALETA, Angélica, de la Caridad, 10400 La Habana (CU); ALVAREZ VERA, Mayling, 10500 La Habana (CU); CASTRO VELAZCO, Jorge, 11600 La Habana (CU); LÓPEZ FERNÁNDEZ, Lázaro, 32500 Artemisa (CU); RAMÍREZ BARTUTIS, Rosa, Liset, 10600 La Habana (CU); PÉREZ FUENTES, Yusleidi, de la Caridad, 10700 La Habana (CU); PÉREZ, GUEVARA, Olga, Lidia, 10500 La Habana (CU); ROMERO FERNÁNDEZ, Yaremy, 17100 La Habana (CU)
(74) Representative: V.O.
(86) International application number: PCT/CU2013/000008
(87) International publication number: WO 2014/101903

(56) References cited:
- WO-A1-2007/031034
- VALDES I ET AL: "A novel fusion protein domain III-capsid from dengue-2, in a highly aggregated form, induces a functional immune response and protection in mice", VIROLOGY, ELSEVIER, AMSTERDAM, NL, vol. 394, no. 2, 25 November 2009 (2009-11-25), pages 249-258, XP026753975, ISSN: 0042-6822, DOI: 10.1016/J.VIROL.2009.08.029 [retrieved on 2009-09-23] cited in the application
- LAURA LAZO ET AL: "A vaccine formulation consisting of nucleocapsid-like particles from Dengue-2 and the fusion protein P64k-domain III from Dengue-1 induces a protective immune response against the homologous serotypes in mice", ACTA TROPICA, vol. 124, no. 2, 1 November 2012 (2012-11-01), pages 107-112, XP055065454, ISSN: 0001-706X, DOI: 10.1016/j.actatropica.2012.06.006
- HOMBACH JOACHIM: "Vaccines against dengue: a review of current candidate vaccines at advanced development stages.", REVISTA PANAMERICANA DE SALUD PÚBLICA = PAN AMERICAN JOURNAL OF PUBLIC HEALTH APR 2007, vol. 21, no. 4, April 2007 (2007-04), pages 254-260, XP002722230, ISSN: 1020-4989 cited in the application
- CLEMENTS D E ET AL: "Development of a recombinant tetravalent dengue virus vaccine: Immunogenicity and efficacy studies in mice and monkeys", VACCINE, ELSEVIER LTD, GB, vol. 28, no. 15, 24 March 2010 (2010-03-24), pages 2705-2715, XP026946252, ISSN: 0264-410X, DOI: 10.1016/J.VACCINE.2010.01.022 [retrieved on 2010-03-08] cited in the application
- GUZMAN MARIA G ET AL: "Domain III of the envelope protein as a dengue vaccine target.", EXPERT REVIEW OF VACCINES FEB 2010, vol. 9, no. 2, February 2010 (2010-02), pages 137-147, XP009177106, ISSN: 1744-8395 cited in the application
- CARLOS L PRG A<3>PEZ ET AL: "In vitro assembly of nucleocapsid-like particles from purified recombinant capsid protein of dengue-2 virus", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 154, no. 4, 21 March 2009 (2009-03-21), pages 695-698, XP019722911, ISSN: 1432-8798

### LISTA DE SEQUENCIAS

<110> Centro de Ingenieria Genética y Biotecnologia
<120> COMPOSICION VACUNAL CONTRA EL VIRUS DENGUE
<130> Dengue-tetravalente
<140> CU 2012-0179
   <141> 27.12.2012
<160> 8
<170> PatentIn Ver. 2.1
<210> 1
   <211> 39
   <212> ADN
   <213> Secuencia Artificial
<220>
   <223> Descripción de la Secuencia Artificial:Oligonucleotido mixto
<400> 1
   atcgactctc gagcgttctc gggggacgat cgtcggggg 39
<210> 2
   <211> 20
   <212> ADN
   <213> Secuencia Artificial
<220>
   <223> Descripción de la Secuencia Artificial:Oligonucleotido K3
<400> 2
   atcgactctc gagcgttctc 20
<210> 3
   <211> 19
   <212> ADN
   <213> Secuencia Artificial
<220>
   <223> Descripción de la Secuencia Artificial:Oligonucleotido 2216
<400> 3
   gggggacgat cgtcggggg 19
<210> 4
   <211> 39
   <212> ADN
   <213> Escherichia coli
<400> 4
   catacctcgc tctgctaatc ctgttaccag tggctgctg 39
<210> 5
   <211> 252
   <212> PRT
   <213> Secuencia Artificial
<220>
   <223> Descripción de la Secuencia Artificial:proteina quimerica DIIIC-1
<400> 5
   Met Gly His His His His His His Gly Ser Arg Leu Lys Met Asp Lys
     1               5                  10                  15
   Leu Thr Leu Lys Gly Met Ser Tyr Val Met Cys Thr Gly Ser Phe Lys
               20                  25                  30
   Leu Glu Lys Glu Val Ala Glu Thr Gln His Gly Thr Val Leu Val Gln
           35                  40                  45
   Val Lys Tyr Glu Gly Thr Asp Ala Pro Cys Lys Ile Pro Phe Ser Thr
       50                  55                  60
   Gln Asp Glu Lys Gly Val Thr Gln Asn Gly Arg Leu Ile Thr Ala Asn
    65                  70                  75                  80
   Pro Ile Val Thr Asp Lys Glu Lys Pro Val Asn Ile Glu Thr Glu Pro
                   85                  90                  95
   Pro Phe Gly Glu Ser Tyr Ile Val Val Gly Ala Gly Glu Lys Ala Leu
               100                 105                 110
   Lys Leu Ser Trp Phe Lys Lys Gly Ser Ser Ile Gly Lys Met Phe Glu
           115                 120                 125
   Ala Thr Ala Arg Gly Ala Arg Arg Met Ala Ile Leu Gly Asp Thr Ala
       130                 135                 140
   Trp Asp Phe Gly Ser Ile Gly Gly Gly Ser Asn Gln Arg Lys Lys Thr
   145                 150                 155                 160
   Ala Arg Pro Ser Phe Asn Met Leu Lys Arg Ala Arg Asn Arg Val Ser
                   165                 170                 175
   Thr Val Ser Gln Leu Ala Lys Arg Phe Ser Lys Gly Leu Leu Ser Gly
               180                 185                 190
   Gln Gly Pro Met Lys Leu Val Met Ala Phe Ile Ala Phe Leu Arg Phe
           195                 200                 205
   Leu Ala Ile Pro Pro Thr Ala Gly Ile Leu Ala Arg Trp Gly Ser Phe
       210                 215                 220
   Lys Lys Ser Gly Ala Ile Lys Val Leu Arg Gly Phe Lys Lys Glu Ile
   225                 230                 235                 240
   Ser Asn Met Leu Asn Ile Met Asn Arg Arg Lys Arg
                   245                 250
<210> 6
   <211> 253
   <212> PRT
   <213> Secuencia Artificial
<220>
   <223> Descripción de la Secuencia Artificial:proteina quimerica DIIIC-2
<400> 6
   Met Gly His His His His His His Gly Ser Arg Leu Arg Met Asp Lys
   1               5                   10                  15
   Leu Gln Leu Lys Gly Met Ser Tyr Ser Met Cys Thr Gly Lys Phe Lys
               20                  25                  30
   Ile Val Lys Glu Ile Ala Glu Thr Gln His Gly Thr Ile Val Ile Arg
           35                  40                  45
   Val Gln Tyr Glu Gly Asp Gly Ser Pro Cys Lys Ile Pro Phe Glu Ile
       50                  55                  60
   Met Asp Leu Glu Lys Arg His Val Leu Gly Arg Leu Ile Thr Val Asn
   65                  70                  75                  80
   Pro Ile Val Thr Glu Lys Asp Ser Pro Val Asn Ile Glu Ala Glu Pro
                   85                  90                  95
   Pro Phe Gly Asp Ser Tyr Ile Ile Ile Gly Val Glu Pro Gly Gln Leu
               100                 105                 110
   Lys Leu Asn Trp Phe Lys Lys Gly Ser Ser Ile Gly Gln Met Phe Glu
           115                 120                 125
   Thr Thr Met Arg Gly Ala Lys Arg Met Ala Ile Leu Gly Asp Thr Ala
       130                 135                 140
   Trp Asp Phe Gly Ser Leu Gly Gly Gly Ser Asn Asn Gln Arg Lys Lys
   145                 150                 155                 160
   Ala Arg Ser Thr Pro Phe Asn Met Leu Lys Arg Glu Arg Asn Arg Val
                   165                 170                 175
   Ser Thr Val Gln Gln Leu Thr Lys Arg Phe Ser Leu Gly Met Leu Gln
               180                 185                 190
   Gly Arg Gly Pro Leu Lys Leu Phe Met Ala Leu Val Ala Phe Leu Arg
           195                 200                 205
   Phe Leu Thr Ile Pro Pro Thr Ala Gly Ile Leu Lys Arg Trp Gly Thr
       210                 215                 220
   Ile Lys Lys Ser Lys Ala Ile Asn Val Leu Arg Gly Phe Arg Lys Glu
   225                 230                 235                 240
   Ile Gly Arg Met Leu Asn Ile Leu Asn Arg Arg Arg Arg
                   245                 250
<210> 7
   <211> 252
   <212> PRT
   <213> Secuencia Artificial
<220>
   <223> Descripción de la Secuencia Artificial:proteina quimerica DIIIC-3
<400> 7
   Met Gly His His His His His His Gly Ser Arg Leu Lys Met Asp Lys
   1               5                   10                  15
   Leu Glu Leu Lys Gly Met Ser Tyr Ala Met Cys Thr Asn Thr Phe Val
               20                  25                  30
   Leu Lys Lys Glu Val Ser Glu Thr Gln His Gly Thr Ile Leu Ile Lys
           35                  40                  45
   Val Glu Tyr Lys Gly Glu Asp Val Pro Cys Lys Ile Pro Phe Ser Thr
       50                  55                  60
   Glu Asp Gly Gln Gly Lys Ala His Asn Gly Arg Leu Ile Thr Ala Asn
   65                  70                  75                  80
   Pro Val Val Thr Lys Lys Glu Glu Pro Val Asn Ile Glu Ala Glu Pro
                   85                  90                  95
   Pro Phe Gly Glu Ser Asn Ile Val Ile Gly Ile Gly Asp Asn Ala Leu
               100                 105                 110
   Lys Ile Asn Trp Tyr Lys Lys Gly Ser Ser Ile Gly Lys Met Phe Glu
           115                 120                 125
   Ala Thr Ala Arg Gly Ala Arg Arg Met Ala Ile Leu Gly Asp Thr Ala
       130                 135                 140
   Trp Asp Phe Gly Ser Val Gly Gly Gly Ser Asn Gln Arg Lys Lys Thr
   145                 150                 155                 160
   Gly Lys Pro Ser Ile Asn Met Leu Lys Arg Val Arg Asn Arg Val Ser
                   165                 170                 175
   Thr Gly Ser Gln Leu Ala Lys Arg Phe Ser Lys Gly Leu Leu Asn Gly
               180                 185                 190
   Gln Gly Pro Met Lys Leu Val Met Ala Phe Ile Ala Phe Leu Arg Phe
           195                 200                 205
   Leu Ala Ile Pro Pro Thr Ala Gly Val Leu Ala Arg Trp Gly Thr Phe
       210                 215                 220
   Lys Lys Ser Gly Ala Ile Lys Val Leu Lys Gly Phe Lys Lys Glu Ile
   225                 230                 235                 240
   Ser Asn Met Leu Ser Ile Ile Asn Lys Arg Lys Lys
                   245                 250
<210> 8
   <211> 252
   <212> PRT
   <213> Secuencia Artificial
<220>
   <223> Descripción de la Secuencia Artificial:proteina quimeica DIIIC-4
<400> 8
   Met Gly His His His His His His Gly Ser Lys Val Arg Met Glu Lys
   1               5                   10                  15
   Leu Arg Ile Lys Gly Met Ser Tyr Thr Met Cys Ser Gly Lys Phe Ser
               20                  25                  30
   Ile Asp Lys Glu Met Ala Glu Thr Gln His Gly Thr Thr Val Val Lys
           35                  40                  45
   Val Lys Tyr Glu Gly Ala Gly Ala Pro Cys Lys Val Pro Ile Glu Ile
       50                  55                  60
   Arg Asp Val Asn Lys Glu Lys Val Val Gly Arg Ile Ile Ser Ser Thr
   65                  70                  75                  80
   Pro Leu Ala Glu Asn Thr Asn Ser Val Thr Asn Ile Glu Leu Glu Pro
                   85                  90                  95
   Pro Phe Gly Asp Ser Tyr Ile Val Ile Gly Val Gly Asn Ser Ala Leu
               100                 105                 110
   Thr Leu His Trp Phe Arg Lys Gly Ser Ser Ile Gly Lys Met Phe Glu
           115                 120                 125
   Ser Thr Tyr Arg Gly Ala Lys Arg Met Ala Ile Leu Gly Glu Thr Ala
       130                 135                 140
   Trp Asp Phe Gly Ser Val Gly Gly Gly Ser Asn Gln Arg Lys Lys Val
   145                 150                 155                 160
   Val Arg Pro Pro Phe Asn Met Leu Lys Arg Glu Arg Asn Arg Val Ser
                   165                 170                 175
   Thr Pro Gln Gly Leu Val Lys Arg Phe Ser Thr Gly Leu Phe Ser Gly
               180                 185                 190
   Lys Gly Pro Leu Arg Met Val Leu Ala Phe Ile Thr Phe Leu Arg Val
           195                 200                 205
   Leu Ser Ile Pro Pro Thr Ala Gly Ile Leu Lys Arg Trp Gly Gln Leu
       210                 215                 220
   Lys Lys Asn Lys Ala Ile Lys Ile Leu Ile Gly Phe Arg Lys Glu Ile
   225                 230                 235                 240
   Gly Arg Met Leu Asn Ile Leu Asn Gly Arg Lys Arg
                   245                 250

## Description

### Technique field

The present invention relates to the field of biotechnology and the pharmaceutical industry, particularly with the obtaining of a vaccine formulation against dengue virus (DV) based on recombinant protein antigens and an oligonucleotide with a defined sequence.

### Background of the previous technique

Dengue fever is a viral disease transmitted by arthropods most widespread affecting human population. Each year they are reported between 50 and 100 million cases of Dengue, 500 000 of them result in the most severe form of the disease, known as Dengue hemorrhagic fever (Guzman et al., Lancet Infect.Dis. 2002; 2:33-42). The causal agent of this disease is the Dengue Virus (DV), belonging to the family *Flaviviridae*, genus *flavivirus.* DV is a viral complex comprising four serotypes. It is an enveloped virus whose lipid membrane contains two of its three structural proteins: the envelope protein and membrane protein. This lipoprotein envelope surrounds the icosahedral nucleocapsid composed by the third of their structural proteins, the capsid protein. (Leyssen et al., Clin.Microbiol.Rev. 2000; 13:67-82).

In the recent decades, the global spread of infections with these viruses has made the development of an effective vaccine, a public health priority. This purpose has been limited by several factors. First of all, infection with one serotype does not induce long-lasting cross protection against the remaining serotypes (Leyssen et al., Clin.Microbiol.Rev. 2000; 13:67-82) and, at the same time, heterotypic secondary infections are the main risk factor for the development of severe forms of the disease (Guzman et al., Lancet Infect.Dis. 2002; 2:33-42; Mongkolsapaya et al., Nat.Med. 2003; 9:921-927). Therefore, an ideal vaccine against DV should induce a long lasting protective immunity against the four viral serotypes (DV1, DV2, DV3 and DV4).

The most advanced vaccine candidates are based on attenuated viral strains through serial passages in cell cultures, or obtained by recombinant way. The viral interference, among the four serotypes in the tetravalent formulations, is the main limitation of this type of candidates making difficult to induce an equivalent functional immune response against the four serotypes; furthermore, they require to be administrated at long intervals between the two or three vaccine doses proposed. (Bhamarapravati et al., Vaccine. 2000; 18:44-47; Kanesa-Thasan et al., Vaccine. 2001; 19:3179-3188; Morrison et al., J.Infect.Dis. 2010; 201:370-377). In addition, due to their nature as live viruses, they cannot be administrated in children less than one year of age.

As an attractive alternative, a series of preclinical studies based on subunit vaccines have been developed. This approach has three key advantages over vaccination with live attenuated virus: 1) they are potentially safe vaccines, 2) the phenomenon of viral interference should not occur due to the non-replicative nature of the immunogen and 3) short vaccination schemes can be proposed, contrary to the administrations of live attenuated virus which require long intervals between vaccine doses to achieve the booster effect.

One of the most promising subunit vaccine candidates, is developed by the company Hawaii Biotech/Merck (Hombach, Rev.Panam.Salud Publica. 2007; 21:254-260). It is a candidate formed by each viral envelope protein from the four serotypes, expressed in insect cells. Monovalent and tetravalent formulations have been assessed in mice and monkeys with immunogenicity results similar to those obtained with the attenuated viruses (Clements et al., Vaccine. 2010; 28:2705-2715). Nevertheless, the monovalent formulations required the addition of potent adjuvants, not licensed for human use, to induce a proper immune response. In turn, the tetravalent formulation assessed in non-human primates contained, not only a non-licensed adjuvant, but also the protein NS1 from DV2, which has some homology with endothelial human cells and consequently, it could provoke an autoimmunity disorder. Additionally, there are no data available about the induction of cell-mediated immunity upon administration of this vaccine candidate, an important arm of the immunity, which has been recently identified as having a protective role against dengue. (Gil et al., Viral Immunol. 2009; 22:23-30; Yauch et al., J.Immunol. 2009; 182:4865-4873; Yauch et al., J.Immunol. 2010; 185:5405-5416).

Keeping the advantages associated with the subunit vaccines, and, at the same time, looking for safer immunogenic formulations containing alum as base adjuvant, the group of Cuban researchers has developed a working line based on the capsid protein and the domain III of the envelope protein of dengue virus (Guzman et al., Exp.Rev.Vaccines. 2010; 9:137-147).

The capsid protein from DV is essential in the virion assembly and protects the viral genome being its main function. Its molecular weight is 9-12 kDa (112-127 amino acids) and it has a basic structure since the 25% of its amino acids are Arginine and Lysine. The protein is located within the virion structure, without exposed regions (Kuhn et al., Cell. 2002; 108:717-725), making it attractive to be included into a vaccine, due to it may not be target of immune-enhancer antibodies. On the other hand, various human CTL epitopes have been identified on its sequence, providing the induction of an effective cell-mediated immunity against the virus (Gagnon et al., J.Virol. 1996; 70:141-147; Gagnon et al., J.Virol. 1999; 73:3623-3629).

Although there are several studies on the structural characteristics of this capsid protein, it was not until the year 2007 that it was evaluated for the first time in terms of immunogenicity in mice. In this study, the capsid from DV2, was obtained as recombinant protein in *Escherichia coli.* Upon a semi purification process, the resultant preparation was assessed in mice, and partial protection after DV2 challenge was obtained without induction of neutralizing antibodies. (Lazo et al., Vaccine. 2007; 25:1064-1070). Later on, purification and *in vitro* aggregation process was established at lab scale, and again, the resultant protein was assessed in mice to measure its functionality in terms of protection (Lopez et al., Arch.Virol. 2009; 154:695-698). The analysis of immunogenicity revealed the induction of cell-mediated immunity measured by secretion of gamma interferon (IFN-y), by the splenocytes of mice receiving the aggregated protein. Such a secretion was dependent on CD4⁺ and CD8⁺ cells. In turn, upon challenge with DV2, a significant protection was obtained in animals immunized with the aggregated protein and such a protection was also dependent on CD4⁺ and CD8⁺ cells (Gil et al., Int.lmmunol. 2009; 21:1175-1183). Based on the aforementioned results, it was proposed to combine, in the same genetic construct, the capsid protein and the Domlll region of the envelope protein, both from DV2. Domlll has been widely described as one receptor-binding region (Chen et al., J.Virol. 1996; 70:8765-8772) and, additionally, it has been reported the induction of neutralizing antibodies and protection in mice immunized with fusion proteins containing this viral region. (Crill et al., J.Virol. 2001; 75:7769-7773; Hermida et al., J.Virol.Methods. 2004; 115:41-49; Simmons et al., Am.J.Trop.Med.Hyg. 2001; 65:159-161). In turn, in non-human primates experiments, it has been demonstrated the induction of a protective immune response only using the Freund's adjuvant (Hermida et al., Vaccine. 2006; 24:3165-3171).

The union viral capsid and the Domllll of the viral envelope protein allows the presence of the two regions potentially protective in a same molecule, capable of simultaneously inducing neutralizing antibodies (Domlll) and cellular immune response (capsid). It was then obtained the genetic construct named DIIIC-2 (Domlll fused to the N-terminus region of the capsid protein, serotype 2), which was expressed in *E*. *coli;* and the resulting protein was purified at lab scale, and underwent the process of aggregation with a mixture of oligonucleotides of unknown sequence. Upon inoculation of three doses in mice, antiviral and neutralizing antibodies were detected. In a similar way, significant IFN-γ secretion was detected in splenocytes from animals immunized with the aggregated protein. Consistently with the cell-mediated immunity, a significant protection upon intracranial challenge was obtained, and such a protection was mediated by CD4⁺ and CD8⁺ cells induced during the immunization process (Valdes et al., Virology. 2009; 394:249-258). Taken together, the aforementioned results allowed selecting the aggregated form of DIIIC-2 for subsequent studies in non-human primates. The first study in non-human primates was accomplished using animals previously infected with DV2, with the main objective to know the booster capacity of DIIIC2. As expected, after administration of DIII-C2, three months after the virus infection, animals developed high levels of antiviral and neutralizing antibodies against the homologous virus, indicating the presence of functional epitopes within the recombinant protein (Valdes et al., Clin.Vaccine Immunol. 2011; 18:455-459).

As a background of this invention, it was known that addition of oligodeoxynucleotides to form aggregate variants of the protein DIIIC-2 favored the cell-mediated immunity and protection against the homologous virus in mice (Valdes et al., Virology. 2009; 394:249-258). Nevertheless, it was unknown whether the sequence can influence on the quality of the induced immune response.

According to the previous referred elements, the development of a vaccine against DV able to induce a safe and effective immune response against the four serotypes is a non-solved problem. The present invention is precisely directed to this objective.

### Explanation of the invention

The present invention solves the aforementioned problem, providing a vaccine composition comprising: a) at least one recombinant antigen comprising amino acids 1 to 99 of the sequence of the capsid protein of dengue virus (DV)and b) the oligodeoxynucleotide identified as SEQ ID NO. 1. In one embodiment of the invention, the recombinant antigen comprising the amino acids 1 to 99 of the capsid protein of dengue virus is a chimeric antigen that is selected from the group consisting of SEQ ID NO. 5 (antigen DIIIC-1), SEQ ID NO. 6 (antigen DIIIC-2), SEQ ID NO. 7 (antigen DIIIC-3) and SEQ ID No. 8 (antigen DIIIC-4).

To demonstrate if the oligonucleotide employed for the protein aggregation influences on the induction of a better immune response, the composition of serotype 2 was selected as a model. The protein DIIIC-2 (chimeric antigen comprising the Domlll of the viral envelope protein and the amino acids 1 to 99 of the capsid protein from DV2) was precipitated in the presence of various oligonucleotides of known sequence, described in the state of the art. It is known that some of these oligonucleotides have adjuvant capacity (Klinman, Int.Rev.Immunol. 2006; 25:1-20; Vollmer, Int.Rev.lmmunol. 2006; 25:125-134). A new oligonucleotide was additionally included in the study, formed by the fusion of two of the mentioned oligonucleotides (Krug et al., Eur.J.Immunol. 2001; 31:2154-2163; Verthelyi et al., J.Immunol. 2001; 166:2372-2377). Upon assessment in mice, we demonstrated that the new oligonucleotide (SEQ ID NO. 1) favored the best cell-mediated immunity, measured by IFN-γ secretion; therefore, it was selected to perform the protection assay using the mouse encephalitis model with the homologous virus. As a result, the DIIIC-2 formulation containing the oligonucleotide of SEQ ID NO. 1 and adjuvanted on alum, elicited a potent protective immune response measured by survival percentage and virus titers in brain.

It is demonstrated herein, for the first time, that the nature of the oligonucleotide is crucial for the induction of a proper cellular immune response, and consequently in the protective capacity of the recombinant protein. Despite trying several oligonucleotides, only one of them, the oligonucleotide whose sequence is identified as SEQ ID NO. 1, turned out to be the best in terms of induction of cellular immune response and protection. Several synthetic oligonucleotides of different sequences were tested, containing or not CpG motifs and having phosphodiester bonds in their structures. This last element differs from oligonucleotides with immunopotentiator activity described in the literature, since links, which are used for the synthesis of these oligonucleotides, are of the type phosphorothioate, in order to protect them from degradation by exonuclease. Additionally, several sizes were tested such as such as 19, 20 and 39 bases. This last 39 bases oligonucleotide contains a number of CpG motifs, and a provision within the sequence, which does not allow including it within the classifications described for oligonucleotides with immnunopotentiator activity in the State of the art.

On the other hand, in all cases these molecules were used for aggregation of recombinant antigens, therefore minimum quantities of them were added. This constitutes another element of difference between the employed oligonucleotides as stimulators of the immune system, as large amounts of them are required to promote that function (Riedl et al., J.Immunol. 2002; 168:4951-4959).

As described above, upon immunological assessment in mice we showed that, unexpectedly, the oligonucleotide whose sequence is identified as SEQ ID NO. 1, significantly potentiated the cellular and protective immune response induced by the recombinant protein DIIIC-2, with differences compared to the rest of the employed oligonucleotides.

Then, the concept in negative to dengue non-human primates was proven. Animals received four doses of aggregated DIIIC-2 formulation with the oligonucleotide of SEQ ID NO. 1, and adjuvanted on alum. Additionally, another group of animals was included in the study, receiving a recombinant antigen comprising amino acids 1-99 of the capsid protein from DV2, previously incubated with the oligonucleotide of SEQ ID NO. 1 and forming nucleocapsid-like particles (NLPs-2).

It was determined the response of antiviral and anti-protein antibodies in addition to the functionality of this response, by the neutralization test, using different strains of DV2 and different cell lines. On the other hand, was also evaluated the cell-mediated immunity by the determination IFN-γ secretion, after the *in vitro* stimulation of peripheral blood mononuclear cells (PBMC) with the infective DV2. Later on, animals were challenged with DV2, and the presence of the virus in the blood was determined. As a result, the protein induced an antibody response with robust neutralizing activity, measured by six different systems (100% seroconversion), as well as a proper cell-mediated immunity, mediated by IFN-γ secretion, before and after challenge with the virus in monkeys. Consistent with the results of immunogenicity, the vaccinated animals were significantly protected against viral challenge, since in two of the three immunized animals no virus was isolated on any day after the challenge, and the third one just showed up one day with viremia values less than 10 plaque forming units per milliliter (pfu/mL).

This study in nonhuman primates, previously negative for dengue, is the first study on the protective ability of a recombinant protein containing the region of the viral capsid DV. This finding allowed to extrapolate these conditions to the other chimeric proteins obtained, corresponding to serotypes 1, 3 and 4.

Next, we designed and obtained the recombinant proteins corresponding to serotypes 1, 3 and 4, which are called DIIIC-1 DIIIC-3 and DIIIC-4, respectively. All molecules were obtained from *E. coli* with appropriate percentages of expression. In turn, these were purified and were recognized by murine polyclonal antibodies specific against the homologous serotype. In addition, it was determined the correct formation of the disulfide bond in each protein, both by mass spectrometry (for DIIIC-1 DIIIC-2, DIIIC-3 and DIIIC-4) and by loss of recognition against murine polyclonal serum upon reduction-carboxymethylation of the cysteines of the Domlll intra-chain disulfide bond (for DIIIC-1 DIIIC-2 and DIIIC-4).

In the studies included in the present application it was demonstrated that the chimeric proteins DIIIC of these serotypes (1, 3 and 4), which are described for the first time, also induce functional and protective immune response in mice against homologous serotypes. Additionally, it is found that the mixture of the four chimeric proteins, previously formulated with the oligonucleotide of SEQ ID NO. 1 and adjuvanted on alum, induces response to all four serotypes, both cellular and humoral, and protective in mice, with no antigenic competition.

The four chimeric proteins DIIIC-1, DIIIC-2, DIIIC-3 and DIIIC-4 were aggregated when adding the oligonucleotide of SEQ ID NO. 1; they were then adyuvanted on alum for further evaluation in mice. After administration of three doses, it was possible to detect the presence of antiviral antibodies against all four serotypes in 100% of the immunized animals. Similarly, neutralizing activity was detected in the sera of these mice, measured against the four serotypes. Consistent with this result, when performing the intracranial challenge with viral serotypes 1 and 4, significant protection was obtained in both cases and therefore, the proof of concept of functionality of monovalent and tetravalent formulations evaluated in mice was demonstrated.

The tetravalent formulation was also capable of inducing a functional immune response, both humoral and cellular, in monkeys. For the evaluation of the immunogenicity of this formulation a second study in dengue-negative nonhuman primates was conducted. The animals received three doses of the tetravalent formulation through different routes, according to the study group, and one month after the last dose, the humoral and cellular immune responses induced were determined. As a result, it was found that 100% of the monkeys induced antiviral immune response of neutralizing antibodies against all the viral serotypes. Also, the cellular immune response test revealed the induction of a positive response in all animals tested.

This work, as a whole, demonstrates the protective ability of the four aggregated proteins DIIIC with the oligonucleotide of SEQ ID NO. 1 against all four serotypes of DV.

The disclosure also comprises a vaccine composition characterized by comprising two of the chimeric antigens which are selected from the group consisting of SEQ ID NO. 5, SEQ ID NO. 6, SEQ ID NO. 7 and SEQ ID No. 8. A further object of the disclosure is a vaccine composition characterized by comprising four chimeric antigens identified as SEQ ID NO. 5, SEQ ID NO. 6, SEQ ID NO. 7 and SEQ ID No. 8.

In another aspect, the invention provides a nucleic acid which is characterized by the sequence identified as SEQ ID NO. 1. As fully shown herein, said nucleic acid is useful for increasing the immune response to a vaccine antigen which comprises at least 50% of the sequence of the capsid protein of DV. In one embodiment of the invention, it is demonstrated the use of the nucleic acid identified as SEQ ID NO.1 to increase the immune response to a recombinant antigen comprising amino acids 1 to 99 of the capsid protein of DV. In a particular embodiment, the use of the nucleic acid identified as SEQ ID NO. 1 to increase the immune response against the chimeric antigens identified as SEQ ID NO. 5, SEQ ID NO. 6, SEQ ID NO. 7 and SEQ ID No. 8 is disclosed.

Additionally, the present invention includes a vaccine composition comprising a) at least one recombinant antigen that comprises amino acids 1 to 99 of the sequence from the capsid of DV and b) the oligonucleotide identified as SEQ ID NO. 1 for use in a method of inducing immune responses against DV. In one aspect, the invention comprises a vaccine composition for use in a method of inducing immune responses against DV, wherein the recombinant antigen comprising amino acids 1 to 99 of said capsid protein , is a chimeric antigen having an amino acid sequence that is selected from the group consisting of SEQ ID NO. 5, SEQ ID NO. 6, SEQ ID NO. 7 and SEQ ID No. 8.

Additionally an aspect of the invention includes the oligonucleotide identified as SEQ ID NO. 1 for use in vaccination against Dengue Virus (DV), wherein the vaccination is with a recombinant antigen comprising amino acids 1 to 99 of the sequence of the capsid protein of DV.

As described herein a study was conducted in mice with the aim of reducing the total dose of each DIIIC protein in the immunization schedule. For this, two different bivalent formulations were administered sequentially, and a third boosting dose was given. As a result, no statistically significant differences between groups tested for any of the four virus serotypes were found, which indicates that it is possible, through sequential administrations of bivalent formulations and booster with tetravalent formulation of DIIIC, comprising the oligonucleotide of SEQ ID NO. 1, to obtain the same levels of immunogenicity that administering three doses of the tetravalent formulation DIIIC, with the oligonucleotide of SEQ ID NO. 1. Therefore also an object of the disclosure comprises the vaccine composition for use in a method of inducing immune responses against DV, where the use comprises the sequential administration of the chimeric antigens identified as SEQ ID NO. 5, SEQ ID NO. 6, SEQ ID NO. 7 and SEQ ID No. 8 in bivalent compositions. The disclosure also comprises the vaccine composition for use in a method to induce immune responses against DV, where said use comprises the further administration of a booster doseof the tetravalent composition of the four chimeric antigens identified as SEQ ID NO. 5, SEQ ID NO. 6, SEQ ID NO. 7 and SEQ ID No. 8.

As shown in the Examples, the compositions comprising at least one antigen that comprises amino acid 1 to 99 of the sequence of the capsid protein of the DV and the oligonucleotide identified as SEQ ID NO. 1 were immunogenic through different routes so that the vaccine composition for use in the method of the invention is administered by the routes that are well known by those skilled people in "the state of art", for example the subcutaneous, the intradermal or intramuscular routes.

### Brief description of the figures

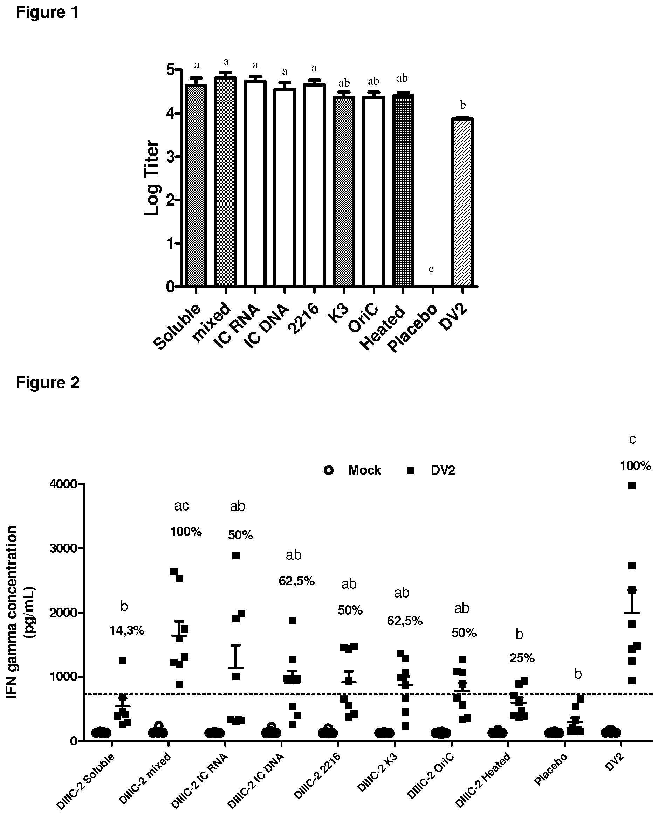

**Figure 1****.** Antiviral antibody response in immunized animals. In the Y-axis the log Titers anti-DV2 is plotted, in the X-axis the groups of animals receiving different variants of formulations DIIIC-2. Titers were determined using an amplified capture ELISA using the virus as an antigen. Statistical analysis was performed using Kruskal-Wallis and multiple comparisons using the Dunn's test *a posteriori.* Different letters indicate statistically significant differences between groups. Data are presented as mean ± standard deviation.
**Figure 2****.** Concentrations of IFN-γ measured by ELISA, after *in vitro* stimulation of splenocytes from mice immunized with different formulations of DIIIC-2 cultured with DV2 or a negative control preparation (mock). On the Y-axis the concentration of IFN-γ (pg/mL) is shown. In the X-axis the groups that received different formulations of DIIIC-2 are represented. Percentages reflected above each set of points represent the percentage of responders. The dashed line indicates the value above which is considered a positive response. Statistical analysis was performed using a single classification ANOVA, and multiple comparisons between groups were performed through the Tukey's test. Different letters indicate statistically significant differences between groups. Data are presented as mean ± standard deviation (n = 8).

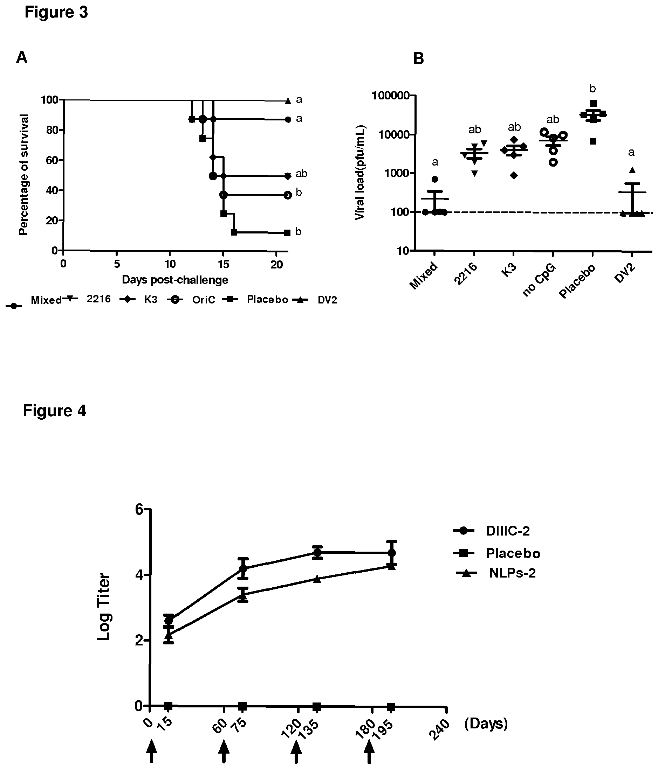

**Figure 3****.** Protection assays against DV2. **A.** Survival curves for immunized mice after intracranial challenge with a lethal strain DV2. The Y-axis represents the percentage of survival and the X-axis represents the observation time after the intracranial challenge. **B.** Viral quantification in VERO cells, measured from infected brain at day 7 post-challenge. The Y-axis represents the number of pfu/mL obtained after plaquing on VERO cells from brain homogenate of infected mouse. The X-axis represents the study groups. For the survival curve, the statistical analysis was performed according to the Log-rank survival test. Different letters indicate statistically significant differences between groups. Data are representative of two independent experiments (n = 10). For viral load, statistical analysis was performed using Kruskal-Wallis and multiple comparisons using the Dunn's test *a posteriori.* Different letters indicate statistical differences between groups. Data are presented as mean ± standard deviation (n = 5). The dashed line indicates the 100 pfu/mL.
**Figure 4****.** Kinetics of anti-DIIIC-2 antibodies generated in monkeys and measured by ELISA. The Y-axis represents the log titer and the X-axis represents time in days during the assay. The black arrows indicate the time of each immunization.

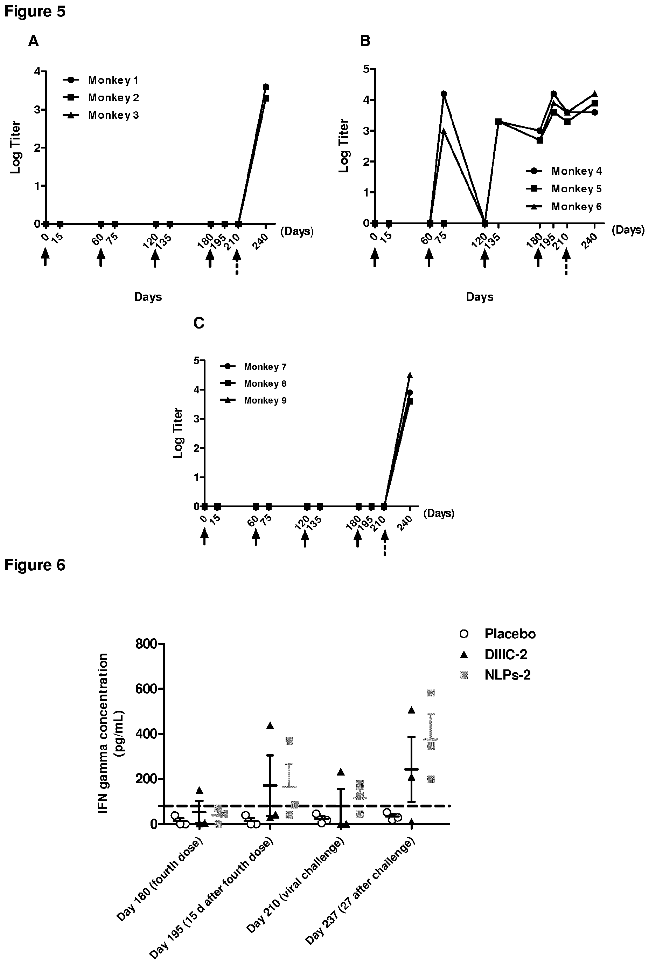

**Figure 5****.** Kinetics of anti-DV antibodies, as measured by a capture ELISA in monkeys immunized with the placebo (**A**), with the aggregated protein DIIIC-2 (**B**) or with the NLPs-2 (**C**). The Y-axis represents the log 1/title, and the X-axis represents time in days during the assay. The black arrows indicate the time of each immunization and the dashed arrow indicates the day of viral challenge.
**Figure 6****.** Concentrations of IFN-γ measured by ELISA after *in vitro* stimulation of peripheral blood mononuclear cells from monkeys, receiving the placebo preparation or DIIIC-2 protein. On the Y-axis the concentration of IFN-γ (pg/mL) is represented and in the X-axis the time of the assay is shown. PBMC from the immunized monkeys were cultured with DV2 or a negative control preparation (mock). Data are presented as mean ± standard deviation (n = 3). The dashed line indicates the value above which is considered a positive response.

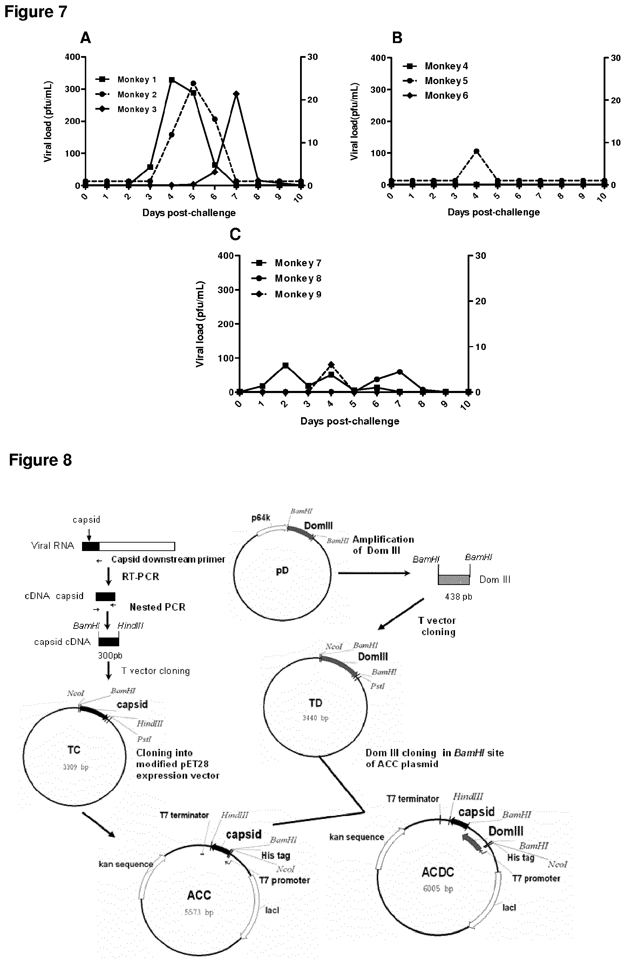

**Figure 7****.** Viral load in the serum of the monkeys immunized with the Placebo formulation (**A**), the aggregated protein DIIIC-2 (**B**) or the NLPs-2 (**C**), measured by direct plaquing on VERO cells. The right axis corresponds to the loads detected in monkeys 2, 5 and 9 (dashed line). The left axis corresponds to the load detected in the remaining animals. On the X-axis the time (in days) after challenge is shown.
**Figure 8****.** Cloning strategy for obtaining the genetic constructs pDIIIC-1 pDIIIC-2, pDIIIC-3 and pDIIIC-4.

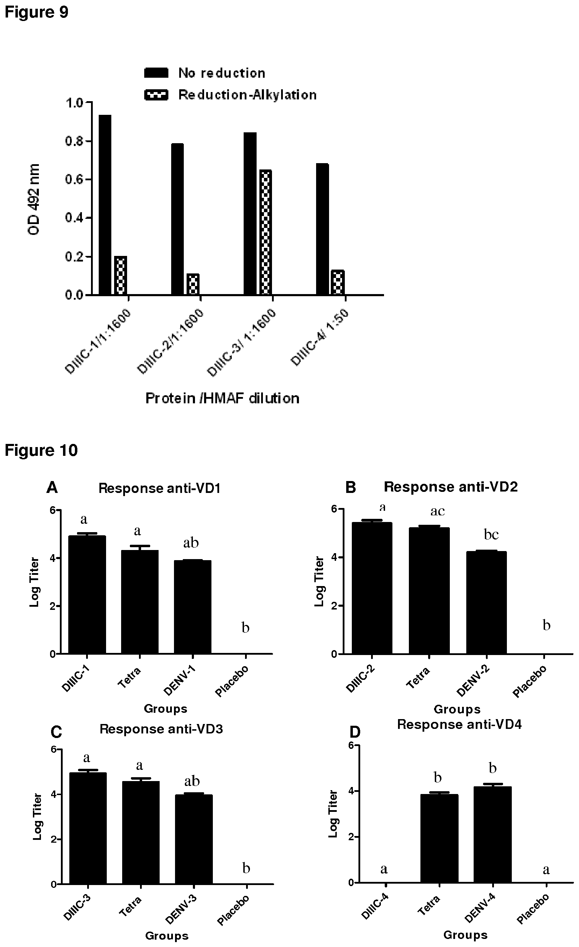

**Figure 9****.** Recognition levels of chimeric proteins DIIIC under reducing and non-reducing conditions by mouse polyclonal antibodies with high neutralizing titers to the homologous serotype, as determined by ELISA. On the Y-axis the absorbance values are plotted, and the X-axis represents the tested proteins.
**Figure 10****.** Antiviral antibody response in immunized animals, measured 15 days after the last immunization. The Y-axis represents the log titer anti-DV, and the X-axis represents the groups of animals receiving each formulation. (A) Anti-DV1 antibody response; (B) anti-DV2 antibody response; (C) anti-DV3 antibody response and (D) anti-DV4 antibody response. Titers were determined using an amplified capture ELISA using each virus as antigen. Statistical analysis was performed using Kruskal-Wallis and multiple comparisons using the Dunn's test *a posteriori.* Different letters indicate statistically significant differences between groups. Data are presented as mean ± standard deviation (n = 10).

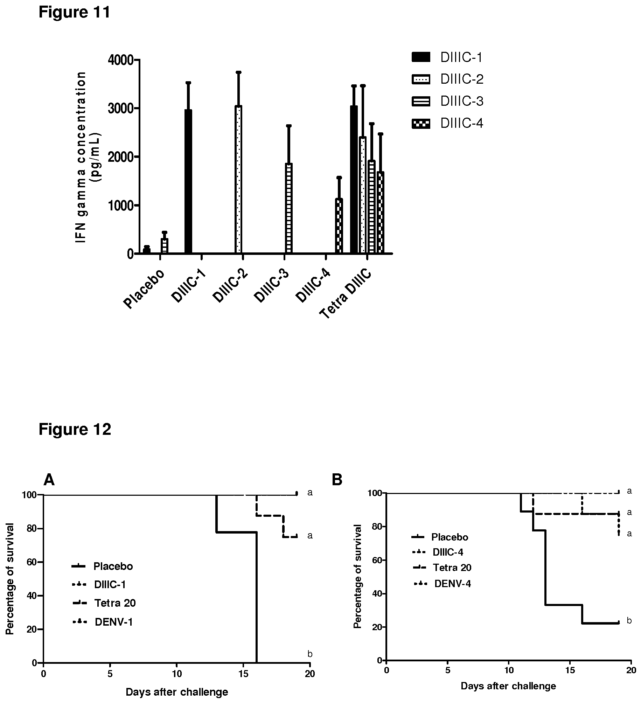

**Figure 11****.** Concentrations of IFN-γ measured by ELISA, after *in vitro* stimulation of splenocytes from mice immunized with monovalent and tetravalent formulations DIIIC (including the oligonucleotide of SEQ ID NO. 1) or with the placebo formulation and stimulated with each protein DIIIC. On the Y-axis the concentration of IFN-γ (pg/mL) is shown. On the X-axis the study groups are represented. Data are presented as mean ± standard deviation.
**Figure 12****.** Survival curves of the immunized mice after intracranial challenge with a lethal strain DV1 (A) and DV4 (B). The Y-axis represents the percentage of survival and the X-axis represents the observation time after the intracranial challenge.
Statistical analysis was performed according to the log-rank survival test. Different letters indicate statistically significant differences between groups. Data are representative of two independent experiments (n = 9).

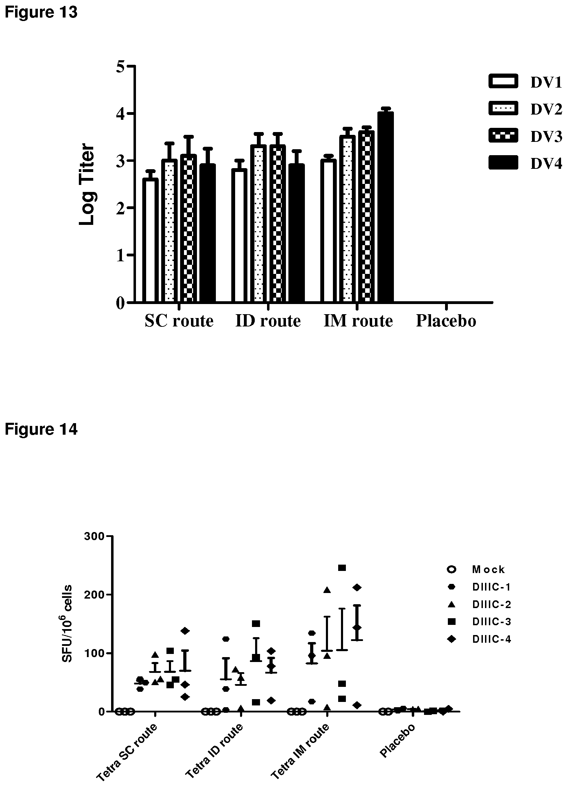

**Figure 13****.** Antiviral antibody response against all four serotypes of DV as determined by a capture ELISA, after three doses of the tetravalent formulation DIIIC with the oligonucleotide of SEQ ID NO. 1 in non-human primates using the subcutaneous (SC), intradermal (ID) and intramuscular (IM) administration route. The Y-axis represents the Log Title anti-DV and the X-axis the groups of animals.
**Figure 14****.** Frequency of cells producing IFN-γ from the peripheral blood lymphocytes stimulated *in vitro* with the recombinant proteins DIIIC after three doses of tetravalent formulation DIIIC with oligonucleotide of SEQ ID NO. 1, in non-human primates. In the Y-axis the frequency of cells producing IFN-γ is represented by the number of spots/mL. On the X-axis the animal groups are represented.

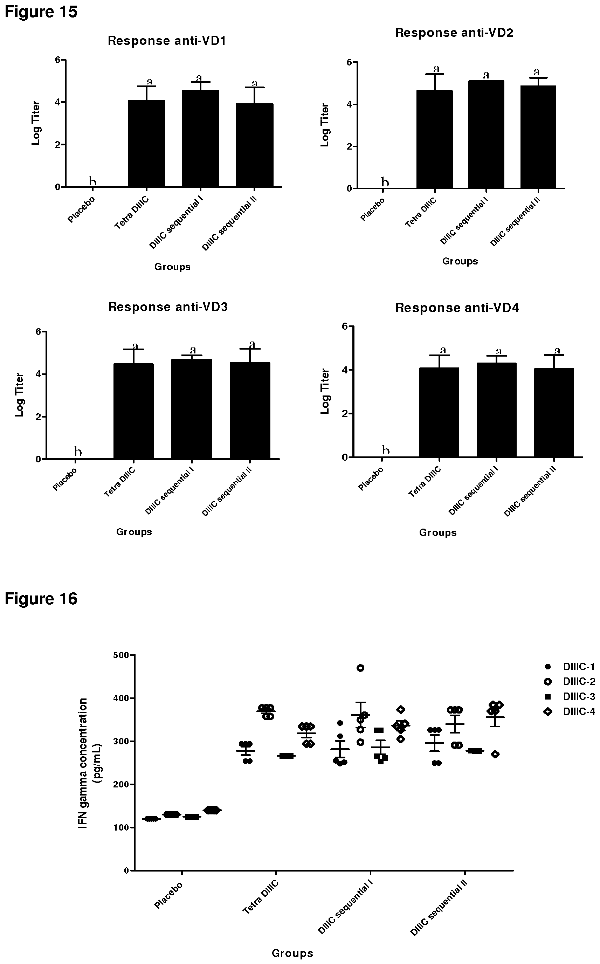

**Figure 15****.** Antiviral antibody response in immunized animals, measured 15 days after the last immunization. The Y-axis represents the log titer anti-DV, and the X-axis the groups of animals receiving each formulation are shown. (**A**) Anti-DV1 antibody response; (**B**) anti-DV2 antibody response; (**C**) anti-DV3 antibody response and (**D**) anti-DV4 antibody response. Titers were determined using an amplified capture ELISA using each virus as antigen. Statistical analysis was performed using Kruskal-Wallis and multiple comparisons using the Dunn's test *a posteriori.* Different letters indicate statistical differences between groups. Data are presented as mean ± standard deviation (n = 10).
**Figure 16****.** Concentrations of IFN-γ measured by ELISA, after *in vitro* stimulation of splenocytes from immunized mice and stimulated with each protein DIIIC. On the Y-axis the concentration of IFN-γ (pg/mL) is shown. On the X-axis the study groups are represented. Data are presented as mean ± standard deviation (n = 5).

### Detailed description of embodiments / examples

### Example 1. Evaluation of the immunogenicity in mice of the aggregated protein DIIIC-2 with different oligonucleotides of defined sequence

Based on the proof of concept in mice with DIIIC-2 protein using a mixture of oligonucleotides of approximately 50 b, of unknown sequence, randomly selected for the aggregation of the protein (Valdes et al, Virology 2009; 394:249-258), various oligonucleotides of defined sequence were tested. Contrary to reports in the literature regarding adjuvant capability of oligonucleotides, those tested in the present study are made only by phosphodiester bonds. Two oligonucleotides of 39 bases were also included, which are not classified within oligonucleotides exerting adjuvant activity, as defined in the literature.

The oligonucleotides tested were:
Oligonucleotide K3 (SEQ ID NO. 2): ATCGACTCT**CG**AG**CG**TTCTC, 20 mer (it contains CpG motifs for humans and monkeys. Backbone of phosphodiester bonds)
Oligonucleotide 2216 (SEQ ID NO. 3): GGGGGA**CG**AT**CG**T**CG**GGGG, 19 mer (it contains CpG motifs for mice and monkeys. Backbone of phosphodiester bonds)
Mixed oligonucleotide (SEQ ID NO. 1): ATCGACTCT**CG**AG**CG**TTCT**CG**GGGGA**CG**AT**CG**T**CG**GGGG, 39 mer (it contains the sequences of oligonucleotides K3 and 2216, backbone of phosphodiester bonds)
Oligonucleotide OriC (SEQ ID NO. 4): CATACCTCGCTCTGCTAATCCTGTTACCAGTGGCTGCTG 39 mer. (backbone of phosphodiester bonds)
Poly I:C backbone of RNA: ICICICICICICICICICICICICIC 26 mer. (backbone of phosphodiester bonds)
Poly I:C backbone of DNA: ICICICICICICICICICICICICIC 26 mer. (backbone of phosphodiester bonds)

The aggregation reaction was conducted with a mass ratio of protein to the mass of the oligonucleotide to allow the precipitation of 50% of the protein in order to have as constant parameter: equal amounts of soluble and aggregated protein in the formulation.

The aggregate protein DIIIC-2 with different oligonucleotides was evaluated in BALB/c mice. The groups were:
Group 1: Soluble DIIIC-2 (non-aggregate and without oligonucleotide) (20 µg protein)
Group 2: DIIIC-2 with mixed oligonucleotide (SEQ ID NO. 1): (20 µg protein + 2 µg oligonucleotide)
Group 3: DIIIC-2 with oligonucleotide poly:IC, ARN (20 µg protein + 2 µg oligonucleotide)
Group 4: DIIIC-2 with oligonucleotide poly:IC, ADN (20 µg protein + 2 µg oligonucleotide)
Group 5: DIIIC-2 with oligonucleotide 2216 (SEQ ID NO. 3) (20 µg protein + 2 µg oligonucleotide)
Group 6: DIIIC-2 with oligonucleotide K3 (SEQ ID NO. 2) (20 µg protein + 2 µg oligonucleotide)
Group 7: DIIIC-2 with oligonucleotide OriC (SEQ ID NO. 4) (20 µg protein + 2 µg oligonucleotide)
Group 8: Heated DIIIC-2 (control of precipitation without oligonucleotide) (it contains half of the precipitated protein (10 µg) and half soluble (10 µg)
Group 9: Placebo with mixed oligonucleotide (SEQ ID NO. 1) (2 µg)
Group 10: 10² pfu of infective DV2

All variants were formulated on alum as adjuvant base and the animals received three doses every 15 days by intraperitoneal route. After the third dose, the detection of reactive antibodies vs. DV2 was determined by a capture ELISA, whereby the titers by end-point dilution of the sera of these animals were determined. As shown in Figure 1, high titers of antiviral antibodies for all groups tested were detected, without statistical difference between them (p>0.05), indicating that aggregation of the chimeric protein DIIIC - 2 does not affect the antiviral antibody response.

With the aim of determining the functionality of the antibodies generated by immunization, the virus neutralization assay *in vitro* was also performed. Table 1 shows the neutralizing titers against DV2. The neutralizing titer was defined as the highest dilution at which 50% reduction in the number of plates is reached. As can be seen, the animals of all groups were positive by neutralization with 100% seroconversion and Geometric Mean Titles (GMT) greater than 1:70.

**Table 1. Development of neutralizing antibodies vs. DV2 in the tested groups**

| **Group** | **1** | **2** | **3** | **4** | **5** | **6** | **7** | **8** | **9** | **10** |
|---|---|---|---|---|---|---|---|---|---|---|
| **GMT** | 153 | 121 | 149 | 73 | 149 | 98 | 107 | 111 | <10 | 69 |

The capacity of aggregated protein DIIIC-2 with different oligonucleotides was also evaluated in this scheme, to generate a cell-mediated immune response. For this, the spleen cells of animals immunized with each variant were extracted and the secretion of IFN- γ in the culture supernatant of splenocytes was measured after stimulation with infectious DV2. Figure 2 shows the values of cytokine secretion by each group and the percentage of responding animals. Although significant levels of secretion were obtained in all groups, only the group immunized with the aggregated protein with the oligonucleotide of SEQ ID NO. 1 protein showed statistically significant differences with respect to the negative control group. Also, in that group 100% of the animals responded positively. Considering both the percentage of responding animals and the mean value of the concentration of IFN- γ in the cellular immune response test, the formulations of aggregated protein DIIIC-2 with oligonucleotides: mixed (SEQ ID NO. 1), 2216 (SEQ ID NO. 3), K3 (SEQ ID NO. 2), and Ori C (SEQ ID NO. 4) were selected for the protection assay .

For this study, the following groups of 15 mice were formed:
Group 1: DIIIC-2 mixed oligonucleotide (SEQ ID NO. 1) (5 µg protein + 0.5 µg oligonucleotide)
Group 2: DIIIC-2 oligonucleotide 2216 (SEQ ID NO. 3) (5 µg protein + 0.5 µg oligonucleotide)
Group 3: DIIIC-2 oligonucleotide K3 (SEQ ID NO. 2) (5 µg protein + 0.5 µg oligonucleotide)
Group 4: DIIIC-2 oligonucleotide OriC (SEQ ID NO. 4) (5 µg protein + 0.5 µg oligonucleotide)
Group 5: Placebo with mixed oligonucleotide (SEQ ID NO. 1) (0.5 µg)
Group 6: 10² pfu of infective DV2

All variants were formulated on alum as adjuvant base and the animals received three doses every 15 days by intraperitoneal route. Two months after the start of the immunization, 10 animals in each group were challenged with 50 median lethal dose (LD₅₀) of homologous neuro-adapted virus, and were observed for 21 days to measure survival. Figure 3A describes the survival percentages obtained. As observed, more than 80% of mice receiving the formulation DIIIC-2 with the oligonucleotide of SEQ ID NO. 1 survived the viral challenge without statistically significant differences compared to the positive control group immunized with the DV2 (p>0.05). In turn, only 10% of animals from the negative control group survived the viral challenge, with statistically significant differences from the group immunized with DIIIC-2 and oligonucleotide of SEQ ID NO. 1, and the positive control (p <0.05). Furthermore, animals immunized with formulations containing oligonucleotides 2216, K3 and OriC reached no survival levels with statistically significant differences compared to the placebo group.

The remaining five animals in each group received 500 LD₅₀ of the same virus, and 7 days after the viral challenge all animals were sacrificed for removal of the brain, and the viral load was measured in VERO cells. Consistent with the survival observed in Figure 3A, mice immunized with the formulation DIIIC-2 with the oligonucleotide of SEQ ID NO. 1 had a low viral load in the brain (<10² pfu/mL), without statistical differences compared to the positive control group (p>0.05), while mice in the group receiving the placebo formulation exhibited a viral load higher than 10⁴ pfu/mL as mean value (Figure 3B). Animals immunized with formulations containing oligonucleotides 2216, OriC and K3 exhibited intermediate levels of viral load between those achieved with the formulation of DIIIC-2 and the oligonucleotide of SEQ ID NO. 1, and the placebo formulation, with no statistically significant differences between them.

### EXAMPLE 2. Proof of concept in nonhuman primates immunized with aggregated protein DIIIC-2 and nucleocapsid-like particles obtained from recombinant capsid protein DV2.

Based on preclinical studies in mice, we evaluated the aggregated protein DIIIC -2 with the oligonucleotide of SEQ ID NO. 1, and adjuvanted with alum in non-human primates negative to DV. In addition, one group receiving the NLPs-2 (containing the oligonucleotide of SEQ ID NO 1.) was evaluated. In turn, the placebo group received a formulation containing the maximum amount of the oligonucleotide of SEQ ID NO. 1, used in the process of aggregation of the recombinant protein adjuvanted on alum. Three animals were included in all groups in the immunization schema.

The selected dose for DIIIC -2 was 100 µg of protein and 10 µg of oligonucleotide of SEQ ID NO. 1, while for the NLPs-2 it was 50 µg of protein and 10 µg of oligonucleotide of SEQ ID NO. 1. Monkeys received four doses subcutaneously, every 2 months. Blood was collected at the time of each dose and fifteen days after, to measure the humoral immune response induced. Figure 4 shows the kinetics of appearance of anti-DIIIC-2 antibodies. As can be seen, in the monkeys that received the formulation DIIIC-2 antibodies began to be detected 15 days after the administration of the first dose. Titers increased following inoculation of the second dose, at values greater than 10,000. After the third dose, titers increased slightly, and after the fourth dose they were kept at the same level (mean mean: 80,000). The response of anti-capsid antibodies in the group immunized with NLPs-2 was similar (Figure 4). Detection of reactive anti-DV2 antibodies was determined by a capture ELISA, whereby the titers by end-point dilution of the sera of these animals were determined. The results of the kinetic study of the development of antiviral antibodies are shown in Figure 5. Antiviral antibodies in the monkeys that received the formulation DIIIC -2 with the oligonucleotide of SEQ ID NO. 1, began to be detected 15 days after administration of the second dose (mean value: 6,000), and fell to undetectable levels at the time of the third dose, i.e., 2 months after receiving the second inoculation of the immunogen. Then, after administration of the third dose, the titers increased to similar levels to those obtained 15 days after the second dose, but at this time they remained detectable (mean: 800) when the fourth dose, which corresponded to 2 months after the third dose. Again, upon receiving the fourth inoculation the animals developed antibodies with values slightly higher than those detected at the time of administration, and these were kept until the time of viral challenge with a mean value of 5,000. When the viral dose was given, it was possible to detect a slight increase in antibody titers at 20 and 27 days after challenge, with mean values of 12,000.

The neutralizing antibody response was also measured in this study, since it represents a possible correlate of protection against this virus. Table 2 shows the values obtained for each sample, at indicated times using the Vero cell line and the strain SB8553 DV2.

As observed, neutralizing antibodies can be detected after administration of the second dose of DIIIC-2. Fifteen days after the third inoculation higher titers were detected, which were kept at the time of the fourth administration. After 15 days, the titers increased, showing a clear booster effect. In turn, one month after the last dose, at the time of viral challenge, the high levels of neutralizing antibodies to all animals immunized with DIIIC-2 were detected. For the group receiving the NLPs-2, as expected, no neutralizing response was detected in any of the times evaluated prior to viral challenge (neutralizing titer less than 10, data not shown.) The placebo group behaved similarly with neutralizing titers less than 10 (data not shown).

**Table 2. Development of neutralizing antibodies vs. DV2 during the immunization scheme in monkeys with DIIIC-2 aggregated protein and the PSNs-2.**

| | **Dose 2** Month 2 | 15 days | **Dose 3** Month 4 | 15 days | **Dose 4** Month 6 | 15 days | **Challenge** Month 7 |
|---|---|---|---|---|---|---|---|
| **DIIIC-2** | <10 | 150.4 | <10 | 620.5 | 207.1 | 888.1 | 1219.8 |
| | <10 | <10 | <10 | 23.6 | 26.2 | 79.8 | 57.7 |
| | <10 | 13 | <10 | 146.2 | 146.5 | 297.7 | 670.3 |
| **GMT** | <10 | 26.9 | <10 | 128.9 | 92.6 | 276.3 | 361.3 |
| **Sero conversion** | **0%** | **66.7%** | **0%** | **100%** | **100%** | **100%** | **100%** |
| **Cell line** | **BHK-21** | | | **Vero** | | **LLC-MK2** | |

One month after the last dose, for the group of DIIIC-2, neutralizing antibodies were also determined, using three viral strains and three different cell lines. In all cases we detected 100% seroconversion, indicating induction of a strong neutralizing response (Table 3).

**Table 3. Neutralizing antibody titers induced by the aggregated protein DIIIC-2 with the oligonucleotide of SEQ ID NO. 1, at the time of challenge, using three different cell lines and viral strains**

| **Strain DV-2** | **A15** | **SB8553** | **WHO** | **SB8553** | **SB8553** |
|---|---|---|---|---|---|
| **DIIIC-2** | 110 | 415.2 | 3861.4 | 1219.8 | 1143.8 |
| | 55 | 14.9 | 169.6 | 57.7 | 62.1 |
| | 150 | 156.2 | 669.7 | 670.3 | 216.1 |
| **GMT** | 96,8 | 98.9 | 759.8 | 361.3 | 248.5 |
| **Seroconversion** | 100% | 100% | 100% | 100% | 100% |

Neutralizing titers of each independent animal are shown, as well as GMT and the percentage of seroconversion achieved with the experimental system used.

The cellular immune response was another of the parameters measured in this study. Peripheral blood lymphocytes, isolated in four points: day of the fourth dose, 15 days after the fourth dose, day of viral challenge, and 27 days after viral challenge, were stimulated with infective DV2, and the secretion of IFN-γ was measured in the culture supernatant. Figure 6 shows the values obtained at each tested point. Of the three animals immunized with DIIIC -2 and the oligonucleotide of SEQ ID NO. 1, one (monkey 6) induced secretion of IFN-γ in the three points discussed, before viral challenge. In turn, after the infection, on day 27, another monkey of this group was also positive, indicating a measurement of an anamnestic cellular response.

Moreover, of the three receiving the NLPs-2, two were positive for IFN-γ on day of viral challenge. In turn, after the infection, on day 27, the three monkeys in this group were positive, indicating in this case also the measurement of an anamnestic cellular response. Importantly, none of the animals receiving the placebo formulation had secretion of antiviral cytokines even after the viral challenge.

To measure protection against DV2, all experimental animals were challenged with an infective dose of the virus, one month after the last dose. The presence of virus in blood was determined by direct measurement in the VERO cell line. Figure 7 shows the results obtained; the animals that received the placebo formulation developed viremia for 3.6 days mean value, and with mean viral load of 10² pfu/mL. Conversely, two of the monkeys receiving the DIIIC-2 protein formulation with the oligonucleotide of SEQ ID NO. 1, did not develop viremia and therefore classified as fully protected.

In one animal (monkey 5), the virus was detected on day 5, and with a very low value of viral load (<10 pfu), which also indicates a significant level of protection.

In the case of the group receiving the NLPs -2, the presence of virus was detected in all monkeys, but the viral load was smaller compared to that detected in the control group. In fact, the monkey 9 exhibited a very low viral load (<10 pfu), also indicating a significant level of protection.

In general, we can say that aggregated proteins based on the viral capsid with the oligonucleotide of SEQ ID NO. 1, induce protective response in monkeys.

### EXAMPLE 3. Obtaining and characterization of proteins: DIIIC-1 DIIIC-3 and DIIIC-4.

The gene fragment BamHI / HindIII containing the DomIII from the envelope protein of DV serotype 1, 3 and 4 (amino acids 286-426) was cloned into the multiple cloning site of the plasmid pET28, fused to the capsid protein of the same virus. This expression vector was previously modified, eliminating the gene sequence between the Ncol and BamHI site, except for the Histidine tag, in order to remove the translation of amino acids not related to the cloned protein. The genetic construct of the plasmids are shown in Figure 8.

In all cases, the proteins are fused to a Histidine tag at its N-terminus, and are expressed under the regulation of the T7 lac promoter. This allowed, once expressed, developing a process of purification of said proteins by combining ionexchange chromatography and affinity chromatography with metal chelates. The same methodology was used for purification of the protein DIIIC-2 previously obtained. Once the four recombinant proteins were purified, we proceeded to characterize them. First, the reactivity of each protein was analyzed versus polyclonal sera from mice immunized with each virus preparation, specifically hyperimmune ascitic fluids anti-DV of each serotype (HMAF). As shown in Figure 9, reactivity was obtained by ELISA of each protein versus HMAF from the homologous viral serotype, indicating a correct presentation of the region Domlll in the context of the capsid, since most of the antibodies generated against viral envelope protein are conformational. Moreover, the state of the disulfide bond (S-S) in the region of Domlll was studied, which must be present in each chimeric molecule. First, it was verified by mass spectrometry each protein, and the correct formation of the bond was verified. Then, based on the fact that the S-S bond is directly involved in the reactivity of Domlll versus polyclonal sera anti-DV, the reactivity of each recombinant protein against the HMAF from the homologous serotype was re-analyzed in reducing and non-reducing conditions. As shown in Figure 9, for DIIIC-1, 2 and 4 proteins, there was a decreased reactivity against homologous HMAF when proteins are irreversibly reduced by alkylation. For DIIIC-3 a decrease is also observed, although less pronounced than in the rest of the proteins. These results confirm the correct formation of S-S bond and its possible role in the reactivity of Domlll against polyclonal anti-DV antibodies.

### EXAMPLE 4. Immunological evaluation of monovalent and tetravalent formulations DIIIC in mice

Each chimeric protein previously aggregated with the oligonucleotide of SEQ ID NO. 1 and the tetravalent mixture of four already aggregated molecules were evaluated in BALB/c mice. All preparations were formulated on alum as adjuvant base, and administered in animals in three doses every 15 days intraperitoneally. As positive controls, four groups immunized with each viral serotype were included. As a negative control, one group received a placebo with the same amount of oligonucleotides contained in the tetravalent formulation, and adjuvanted on alum. The amounts of protein for the tetravalent formulation were 20 µg each and 8 µg of total oligonucleotide of SEQ ID NO. 1. Monovalent formulations contained 20 µg of protein and 2 µg of oligonucleotide of SEQ ID NO. 1.

After the third dose, the detection of antibodies reactive with each virus was determined by a capture ELISA (Figure 10), through which the titers were determined by end-point dilution of the sera of these animals. Animals receiving monovalent formulations had high titers of anti-viral antibodies versus serotypes 1, 2 and 3, whereas animals immunized with DIIIC-4, had no titers against the homologous serotype (DV4). However, animals receiving the tetravalent formulation Tetra-DIIIC developed antibodies against DV4, but with lower titers compared to the other serotypes. This result indicates that the mixture of the four proteins favors the induction of antibodies to DV4, possibly through some level of cross reactivity with the other serotypes.

With the aim of determining the functionality of the antibodies generated by immunization with the tetravalent formulation Tetra-DIIIC, the virus neutralization assay *in vitro* (Table 4) against the serotype 1, 2, 3 and 4 was carried out. The assay was performed following the guidelines of the World Health Organization (WHO), and using reference strains. Mice immunized with DIIIC-1, 2 and 3 proteins exhibited high titers of neutralizing antibodies against homologous virus, comparable to those elicited by the replicative virus in mice of the control groups. Animals immunized with the DIIIC-4 protein did not show the response, where consistent with the results of the antiviral response, no neutralizing antibodies were detected against DV4. However, the tetravalent formulation, in addition to induce neutralizing antibodies against the serotypes 1, 2 and 3, it also induced functional antibodies against DV4.

**Table 4. Neutralizing antibody titers in mice immunized with monovalent and tetravalent formulations of the chimeric proteins DIIIC.**

| | **DV to neutralize** | | | |
|---|---|---|---|---|
| **Groups** | **DV1** | **DV2** | **DV3** | **DV4** |
| DIIIC-1 | >500 | - | - | - |
| DIIIC-2 | - | >500 | - | - |
| DIIIC-3 | - | - | >500 | - |
| DIIIC-4 | - | - | - | <10 |
| Tetra DIIIC | 347.1 | >1000 | >1000 | 166.34 |
| Control DV1 | 188.6 | - | - | - |
| Control DV2 | - | >500 | - | - |
| Control DV3 | - | - | >500 | - |
| Control DV4 | - | - | - | >250 |

In all cases a mixture of sera from the mice were analyzed. (-): Not determined.

The cellular immune response was another of the parameters measured in this study. For this, the spleen cells of animals immunized with the tetravalent formulation Tetra-DIIIC 30 days after the last dose were extracted and secretion of IFN- γ in the culture supernatant of splenocytes was measured after stimulation with the four recombinant proteins. As negative control, the splenocytes of mice inoculated with the placebo formulation were used. The results obtained are shown in Figure 11. As observed, high levels of IFN-γ were detected after stimulation with the four recombinant proteins, indicating equivalence in the cellular response obtained to each serotype.

Finally, the protection assay was performed on the model of viral encephalitis in mice. For this experiment, animals immunized with a tetravalent formulation Tetra-DIIIC, monovalent formulations DIIIC-1 and DIIIC -4, the positive control animals (immunized with DV1 and DV4, respectively) and those immunized with the placebo formulation were selected. In turn, the challenge viruses used were DV1 and DV4, capable of causing the death of animals. As shown in Figure 12, high levels of protection was obtained against serotypes 1 and 4, both with monovalent and tetravalent formulations, in all cases with no statistical differences compared to the viral control group (p>0.05). These results show the protective ability of the formulations tested against serotypes 1 and 4, based on the aggregated proteins DIIIC with the oligonucleotide of SEQ ID NO. 1.

### EXAMPLE 5. Immunological evaluation in non-human primates of the tetravalent formulation Tetra-DIIIC

The tetravalent formulation Tetra DIIIC evaluated in mice was similarly assessed in non-human primates. Three study groups of three animals each were formed to receive the tetravalent formulation through three different routes of antigen administration: Group 1: subcutaneous, Group 2: Intradermal and Group 3: intramuscular. Groups 1 and 3 were given 50 µg of each chimeric protein, previously aggregated with 5 µg of oligonucleotide of SEQ ID NO. 1; all mixed and adjuvanted on alum. Group 2 received 10 times less immunogen by intradermal route than the other two routes; it was 5 µg of each protein and 2 µg of total oligonucleotide of SEQ ID NO. 1, all mixed and adjuvanted on alum. The placebo group received the same amount of oligonucleotide of SEQ ID NO. 1 than the groups 1 and 3, adjuvanted on alum and given intramuscularly. Blood was collected at the time of each dose, and one month after them to measure the humoral and cellular immune response induced.

Detection of reactive antibodies to each viral serotype was determined by capture ELISA, whereby the titers by end-point dilution of the sera of these animals were determined. Figure 13 shows the antiviral antibody response generated in monkeys after three doses of the Tetra-DIIIC formulation including the oligonucleotide of SEQ ID NO. 1. As observed, regardless of the route of antigen administration, monkeys elicited an antibody response capable of recognizing the four viral serotypes in the capture ELISA system. In addition, this response showed a pattern of homogeneity for the four DV; being this an important step toward the development of a vaccine against this human pathogen, which requires a balanced immune response.

The measurement of neutralizing antibodies was performed using the technique of plate reduction neutralization test (PRNT) in VERO cells, and using the viral strains Jamaica DV1, SB8553 DV2, Nicaragua DV3 and Dominica DV4. The values obtained after the third immunization are shown in Table 5.

**Table 5. Titers of neutralizing antibodies in non-human primates immunized with the tetravalent formulation including Tetra DIIIC with the oligonucleotide of SEQ ID NO. 1.**

| | **DV to neutralize** | | | |
|---|---|---|---|---|
| **Route** | **DV1** | **DV2** | **DV3** | **DV4** |
| **Subcutaneous** | 77.4 | 215.9 | 146.5 | 46.7 |
| | 253.7 | 186.9 | 138.3 | 102 |
| | 88.5 | 192.9 | 668.1 | 86.1 |
| **GMT** | **120.2** | **198.2** | **238.3** | **74.3** |
| **Intradermal** | 57.1 | 153.1 | 617.8 | 35.6 |
| | 44.6 | 184.3 | 165.2 | 42.5 |
| | 49.3 | 88.6 | 200 | 494.2 |
| **GMT** | **50.1** | **135.7** | **273.3** | **90.8** |
| **Intramuscular** | 247.2 | 472.7 | 254.3 | 113.1 |
| | 47.7 | 111.4 | 390.3 | 121.6 |
| | 231.6 | 215.9 | 577.5 | 798.5 |
| **GMT** | **139.8** | **224.9** | **385.6** | **222.3** |

All animals immunized with the formulation Tetra DIIIC generated an antibody response capable of neutralizing viral infection *in vitro,* regardless of the route of antigen administration. Generating a neutralizing antibody response with 100% of animals responding to all four serotypes is currently a premise on the development of a vaccine against dengue. In the placebo group, the neutralizing titers were lower than 20 in all immunized animals.

The cellular immune response was one of the measured parameters. The PBMCs were stimulated with each of the recombinant proteins DIIIC, and the frequency of cells producing IFN-γ was measured by ELISPOT assay. Figure 14 shows the values obtained. Following the administration of three doses of tetravalent formulation Tetra-DIIIC, animals generated cells capable of secreting the antiviral cytokine, to stimulation with the recombinant proteins, the response being relatively greater in animals immunized intramuscularly. In all groups evaluated there was a 100% of animals responding to the four proteins tested.

### EXAMPLE 6. Immunological evaluation in mice of the combined administration of the bivalent and tetravalent formulations of proteins DIIIC and oligonucleotide of SEQ ID NO. 1, in prime-boost regime

With the purpose of only giving two doses of each protein formulation DIIIC in the same immunization scheme, the following experimental design was carried out:
Sequential Group I dose 1: Bivalent formulation DIIIC-1/DIIIC-2
   dose 2: Bivalent formulation DIIIC-3/DIIIC-4
   dose 3: Tetravalent formulation Tetra DIIIC
Sequential Group II dose 1: Bivalent formulation DIIIC-1/DIIIC-3
   dose 2: Bivalent formulation DIIIC-2/DIIIC-4
   dose 3: Bivalent formulation Tetra DIIIC
Group Tetra DIIIC: All doses of tetravalent formulation Tetra DIIIC

Chimeric proteins DIIIC, previously aggregated with the oligonucleotide of SEQ ID NO. 1, were mixed to form both bivalent and tetravalent formulations. The immunogen comprised 20 µg of each protein and 4 µg of oligonucleotide of SEQ ID NO. 1, per bivalent formulation. The amounts of protein for the tetravalent formulation were 20 µg each and 8 µg of total oligonucleotide of SEQ ID NO. 1. All variants were formulated on alum as adjuvant, and were administered every 15 days intraperitoneally. The group that received only the tetravalent formulation Tetra-DIIIC was used as positive control and the placebo group as a negative control.

After the third dose, the detection of antibodies reactive with each virus was determined by a capture ELISA (Figure 15), through which the titers by end-point dilution of the sera of these animals were determined. As a result, we found that there were no statistically significant differences between the tested groups for any of the four viral serotypes, indicating that is possible, through sequential administrations of bivalent formulations and booster with the tetravalent Tetra-DIIIC, to obtain the same levels of immunogenicity that administering three doses with the tetravalent formulation.

Additionally, the cellular immune response was also measured. Spleen cells from animals immunized from each group of the study were extracted 30 days after the last dose, and the secretion of IFN- γ in the culture supernatant of splenocytes was measured after stimulation with the four recombinant proteins. As negative control, the splenocytes of mice inoculated with the placebo formulation were used. The results obtained are shown in Figure 16. As can be seen, high levels of IFN-γ were detected after stimulation with the four recombinant proteins in all groups (except for the placebo), indicating equivalence in the cellular response obtained, regardless of the immunization regime used and bivalent formulations administered in the first two doses.

## Claims

1. Vaccine composition **characterized in that** it comprises a) at least one recombinant antigen comprising amino acids 1 to 99 of the sequence of the capsid protein of dengue virus (DV) and b) the oligonucleotide identified as SEQ ID NO. 1.

2. The vaccine composition of claim 1 wherein the recombinant antigen comprising amino acids 1 to 99 of the sequence of the capsid protein of dengue virus (DV) is a chimeric antigen that is selected from the group consisting of SEQ ID NO. 5 (antigen DIIIC-1), SEQ ID NO. 6 (antigen DIIIC-2), SEQ ID NO. 7 (antigen DIIIC-3), and SEQ ID No. 8 (antigen DIIIC- 4).

3. The vaccine composition of claim 1 which is **characterized by** comprising two of the chimeric antigens which are selected from the group consisting of SEQ ID NO. 5, SEQ ID NO. 6, SEQ ID NO. 7 and SEQ ID No. 8.

4. The vaccine composition of claim 1 which is **characterized by** comprising four chimeric antigens identified as SEQ ID NO. 5, SEQ ID NO. 6, SEQ ID NO. 7 and SEQ ID No. 8.

5. Nucleic acid identified as SEQ ID NO. 1.

6. The nucleic acid identified as SEQ ID NO. 1 for use in increasing the immune response to a vaccine antigen, wherein said antigen comprises a recombinant antigen comprising amino acids 1 to 99 of the sequence of the capsid protein of dengue virus (DV).

7. Nucleic acid for use according to claim 6 wherein the recombinant antigen comprising amino acids 1 to 99 of the capsid protein of dengue virus (DV) is a chimeric antigen, which is selected from the group consisting of SEQ ID NO. 5, SEQ ID NO. 6, SEQ ID NO. 7 and SEQ ID No. 8.

8. A vaccine composition comprising a) at least one recombinant antigen comprising amino acids 1 to 99 of the sequence of the capsid protein of dengue virus (DV) and b) the oligonucleotide identified as SEQ ID NO. 1, for use in the induction of immune responses against dengue virus (DV).

9. The vaccine composition for use according to claim 8 wherein the recombinant antigen comprising amino acids 1 to 99 of the sequence of the capsid protein of dengue virus (DV), is a chimeric antigen having an amino acid sequence that is selected from the group consisting of SEQ ID NO. 5, SEQ ID NO. 6, SEQ ID NO. 7 and SEQ ID No. 8.

10. The vaccine composition for use according to claim 9, wherein said use comprises the sequential administration of the chimeric antigens identified as SEQ ID NO. 5, SEQ ID NO. 6, SEQ ID NO. 7 and SEQ ID No. 8 in bivalent compositions.

11. The vaccine composition for use according to claim 10, wherein said use comprises the further administration of a booster dose of the tetravalent composition, comprising the four chimeric antigens identified as SEQ ID NO. 5, SEQ ID NO. 6, SEQ ID NO. 7 and SEQ ID No. 8.

12. The vaccine composition for use according to claim 8 wherein said use comprises the administration of the vaccine composition comprising a) at least one recombinant antigen comprising amino acids 1 to 99 of the sequence of the capsid protein of DV and b) the oligonucleotide identified as SEQ ID NO. 1, by subcutaneous, intradermal or intramuscular route.

## Patentansprüche

1. Impfstoffzusammensetzung, **dadurch gekennzeichnet, dass** sie a) mindestens ein rekombinantes Antigen umfasst, umfassend die Aminosäuren 1 bis 99 der Sequenz des Kapsidproteins des Dengue-Virus (DV), und b) das Oligonukleotid, identifiziert als SEQ ID NR. 1.

2. Impfstoffzusammensetzung nach Anspruch 1, wobei das rekombinante Antigen, umfassend Aminosäuren 1 bis 99 der Sequenz des Kapsidproteins des Dengue-Virus (DV), ein chimäres Antigen ist, ausgewählt aus der Gruppe, bestehend aus SEQ ID NR. 5 (Antigen DIIIC-1), SEQ ID NR. 6 (Antigen DIIIC-2), SEQ ID NR. 7 (Antigen DIIIC-3) und SEQ ID Nr. 8 (Antigen DIIIC-4).

3. Impfstoffzusammensetzung nach Anspruch 1, **dadurch gekennzeichnet, dass** sie zwei der chimären Antigene umfasst, die aus der Gruppe ausgewählt sind, bestehend aus SEQ ID NR. 5, SEQ ID NR. 6, SEQ ID NR. 7 und SEQ ID Nr. 8.

4. Impfstoffzusammensetzung nach Anspruch 1, **dadurch gekennzeichnet, dass** sie vier als SEQ ID NR. 5, SEQ ID NR. 6, SEQ ID NR. 7 und SEQ ID Nr. 8 identifizierte chimäre Antigene umfasst.

5. Nukleinsäure, identifiziert als SEQ ID NR. 1.

6. Nukleinsäure, identifiziert als SEQ ID NR. 1 zur Verwendung bei der Steigerung der Immunantwort auf ein Impfstoff-Antigen, wobei das Antigen ein rekombinantes Antigen umfasst, umfassend die Aminosäuren 1 bis 99 der Sequenz des Capsidproteins des Dengue-Virus (DV).

7. Nukleinsäure zur Verwendung nach Anspruch 6, wobei das rekombinante Antigen, umfassend die Aminosäuren 1 bis 99 des Kapsidproteins des Dengue-Virus (DV), ein chimäres Antigen ist, ausgewählt aus der Gruppe, bestehend aus SEQ ID NR. 5, SEQ ID NR. 6, SEQ ID NR. 7 und SEQ ID Nr. 8.

8. Impfstoffzusammensetzung, umfassend a) mindestens ein rekombinantes Antigen, umfassend Aminosäuren 1 bis 99 der Sequenz des Kapsidproteins des Dengue-Virus (DV), und b) das Oligonukleotid, identifiziert als SEQ ID NO 1, zur Verwendung bei der Induktion von Immunantworten gegen Dengue-Virus (DV).

9. Impfstoffzusammensetzung zur Verwendung nach Anspruch 8, wobei das rekombinante Antigen, umfassend Aminosäuren 1 bis 99 der Sequenz des Capsidproteins des Dengue-Virus (DV), ein chimäres Antigen mit einer Aminosäuresequenz ist, die ausgewählt ist aus der Gruppe bestehend aus SEQ ID NR. 5, SEQ ID NR. 6, SEQ ID NR. 7 und SEQ ID Nr. 8.

10. Impfstoffzusammensetzung zur Verwendung nach Anspruch 9, wobei die Verwendung die sequentielle Verabreichung der chimären Antigene, identifiziert als SEQ ID NR. 5, SEQ ID NR. 6, SEQ ID NR. 7 und SEQ ID Nr. 8, in zweiwertigen Zusammensetzungen umfasst.

11. Impfstoffzusammensetzung zur Verwendung nach Anspruch 10, wobei die Verwendung die weitere Verabreichung einer Auffrischungsdosis der vierwertigen Zusammensetzung umfasst, umfassend die vier als SEQ ID NR. 5, SEQ ID NR. 6, SEQ ID NR. 7 und SEQ ID Nr. 8 identifizierten chimären Antigene.

12. Impfstoffzusammensetzung zur Verwendung nach Anspruch 8, wobei die Verwendung die Verabreichung der Impfstoffzusammensetzung umfasst, umfassend a) mindestens ein rekombinantes Antigen, umfassend Aminosäuren 1 bis 99 der Sequenz des Kapsidproteins von DV und b) das Oligonukleotid, identifiziert als SEQ ID NR. 1 auf subkutanem, intradermalem oder intramuskulärem Weg.

## Revendications

1. Composition de vaccin **caractérisée en ce qu'**elle comprend:
a) au moins un antigène recombinant comprenant 1 à 99 acides aminés de la séquence de la protéine de capside du virus de la dengue (DV), et
b) l'oligonucléotide identifié comme SEQ ID NO: 1.

2. La composition de vaccin selon la revendication 1, dans laquelle l'antigène recombinant comprenant 1 à 99 acides aminés de la séquence de la protéine de capside du virus de la dengue (DV) est un antigène chimérique qui est choisi dans le groupe consistant en SEQ ID NO: 5 (antigène DIIIC-1), SEQ ID NO: 6 (antigène DIIIC-2), SEQ ID NO: 7 (antigène DIIIC-3) et SEQ ID NO: 8 (antigène DIIIC-4).

3. La composition de vaccin selon la revendication 1, qui est **caractérisée en ce qu'**elle comprend deux des antigènes chimériques qui sont choisis dans le groupe consistant en SEQ ID NO: 5, SEQ ID NO: 6, SEQ ID NO: 7 et SEQ ID NO: 8.

4. La composition de vaccin selon la revendication 1, **caractérisée en ce qu'**elle comprend quatre antigènes chimériques identifiés comme étant SEQ ID NO: 5, SEQ ID NO: 6, SEQ ID NO: 7 et SEQ ID NO: 8.

5. Acide nucléique identifié comme étant SEQ ID NO: 1.

6. L'acide nucléique identifié comme étant SEQ ID NO:1 pour utilisation dans l'augmentation de la réponse immunitaire à un antigène de vaccin, dans lequel ledit antigène comprend un antigène recombinant comprenant 1 à 99 acides aminés de la séquence de la protéine de capside du virus de la dengue (DV).

7. Acide nucléique pour utilisation selon la revendication 6, dans laquelle l'antigène recombinant comprenant 1 à 99 acides aminés de la protéine de capside du virus de la dengue (DV) est un antigène chimérique, qui est choisi dans le groupe constitué de SEQ ID NO: 5, SEQ ID NO: 6, SEQ ID NO: 7 et SEQ ID NO: 8.

8. Une composition de vaccin comprenant:
a) au moins un antigène recombinant comprenant 1 à 99 acides aminés de la séquence de la protéine de capside du virus de la dengue (DV), et
b) l'oligonucléotide identifié comme étant SEQ ID NO: 1 pour utilisation dans l'induction de réponses immunitaires contre le virus de la dengue (DV).

9. La composition de vaccin pour utilisation selon la revendication 8, dans laquelle l'antigène recombinant comprenant 1 à 99 acides aminés de la séquence de la protéine de capside du virus de la dengue (DV), est un antigène chimérique, ayant une séquence d'acides aminés qui est choisie dans le groupe consistant en SEQ ID NO: 5, SEQ ID NO: 6, SEQ ID NO: 7 et SEQ ID NO: 8.

10. La composition de vaccin pour utilisation selon la revendication 9, dans laquelle la dite utilisation comprend l'administration séquentielle dans des compositions bivalentes des antigènes chimériques identifiés en tant que SEQ ID NO: 5, SEQ ID NO: 6, SEQ ID NO: 7 et SEQ ID NO: 8.

11. La composition de vaccin pour utilisation selon la revendication 10, dans laquelle ladite utilisation comprend l'administration supplémentaire d'une dose de rappel de la composition tétravalente, comprenant les quatre antigènes chimériques identifiés comme étant SEQ ID NO: 5, SEQ ID NO: 6, SEQ ID NO: 7 et SEQ ID NO: 8.

12. La composition de vaccin pour utilisation selon la revendication 8, dans laquelle ladite utilisation de la composition de vaccin comprend:
a) au moins un antigène recombinant comprenant 1 à 99 acides aminés de la séquence de la protéine de capside du virus de la dengue (DV) et
b) l'oligonucléotide identifié comme étant SEQ ID NO: 1, est administrée par voie sous-cutanée, intradermique ou intramusculaire.
